(19) Europäisches Patentamt
European Patent Office
Office européen des brevets

(11) Veröffentlichungsnummer: 0 342 440
A1

# (12) EUROPÄISCHE PATENTANMELDUNG

(21) Anmeldenummer: 89108066.5

(22) Anmeldetag: 04.05.89

(51) Int. Cl.4: C07C 149/273, C07C 43/29, C07C 43/295, C07C 59/68, C07C 69/736, C07C 155/08, C07C 147/00, A01N 31/00

(30) Priorität: 17.05.88 DE 3816701
21.01.89 DE 3901706

(43) Veröffentlichungstag der Anmeldung:
23.11.89 Patentblatt 89/47

(84) Benannte Vertragsstaaten:
BE CH DE ES FR GB IT LI NL SE

(71) Anmelder: BAYER AG

D-5090 Leverkusen 1 Bayerwerk(DE)

(72) Erfinder: Busse, Ulrich, Dr.
Am Bandenfeld 106
D-5657 Haan(DE)
Erfinder: Kirsten, Rolf, Dr.
Carl-Langhans-Strasse 27
D-4019 Monheim(DE)
Erfinder: Santel, Hans-Joachim, Dr.
Gruenstrasse 9 a
D-5090 Leverkusen 1(DE)
Erfinder: Schmidt, Robert R., Dr.
Im Waldwinkel 110
D-5060 Bergisch Gladbach 2(DE)
Erfinder: Strang, Harry, Dr.
Unterdorfstrasse 6 a
D-4000 Düsseldorf 31(DE)

## (54) Halogensubstituierte Phenoxybenzyl (thi)ol-Derivate.

(57) Die Erfindung betrifft neue halogensubstituierte Phenoxybenzyl(thi)ol-Derivate der allgemeinen Formel (I)

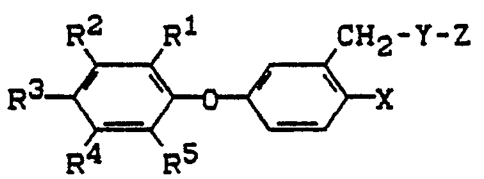

(I)

(worin X für Halogen steht und die Reste $R^1$-$R^5$, Y und Z die in der Beschreibung genannten Bedeutungen haben), Verfahren zu deren Herstellung und deren Verwendung als Herbizide.

EP 0 342 440 A1

## Halogensubstituierte Phenoxybenzyl(thi)ol-Derivate

Die Erfindung betrifft neue halogensubstituierte Phenoxybenzyl(thi)ol-Derivate, Verfahren zu ihrer Herstellung und ihre Verwendung als Herbizide.

Es ist bereits bekannt, daß bestimmte Phenoxybenzoesäure-Derivate, wie z.B. 3-(2,4-Dichlor-phenoxy)-6-nitro-benzoesäure-methylester (Bifenox) herbizid wirksam sind (vgl. US-P 3 652 645 und US-P 3 776 715). Die Wirkung dieser bekannten Verbindungen ist jedoch nicht immer zufriedenstellend.

Es wurden nun neue halogensubstituierte Phenoxybenzyl(thi)ol-Derivate der allgemeinen Formel (I)

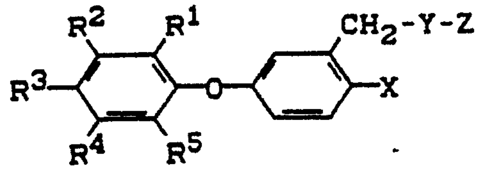

(I)

in welcher

$R^1$ für Wasserstoff, Halogen, Cyano oder Trifluormethyl steht,
$R^2$ für Wasserstoff oder Halogen steht,
$R^3$ für Halogen, Cyano, Trifluormethyl, Trifluormethoxy oder Trifluormethylsulfonyl steht,
$R^4$ für Wasserstoff oder Halogen steht,
$R^5$ für Wasserstoff oder Halogen steht,
- mit der Maßgabe, das wenigstens zwei der Substituenten $R^1$, $R^2$, $R^4$ und $R^5$ von Wasserstoff verschieden sind -
X für Halogen steht,
Y für Sauerstoff, Schwefel, Sulfinyl oder Sulfonyl steht und
Z für Wasserstoff, für gegebenenfalls durch Cyano, Halogen, Hydroxy, Alkoxy, Alkylthio, Carboxy, Alkoxycarbonyl, Alkylcarbonyloxy oder Alkoxycarbonylalkoxycarbonyl substituiertes Alkyl, für jeweils gegebenenfalls durch Halogen und/oder Alkyl substituiertes Cycloalkyl oder Cycloalkylalkyl, für jeweils gegebenenfalls durch Halogen substituiertes Alkenyl oder Alkinyl, oder für eine der nachstehenden Gruppierungen steht

$-(CH_2)_m-Z^1$, 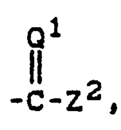, 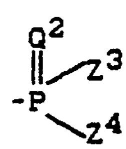

worin
m für für die Zahlen 0, 1, 2 oder 3 steht,
$Q^1$ und $Q^2$ jeweils für Sauerstoff oder Schwefel stehen,
$Z^1$ für jeweils gegebenenfalls durch Halogen, Cyano, Nitro, Alkyl, Halogenalkyl, Alkoxy, Halogenalkoxy, Alkylthio, Halogenalkylthio, Dialkylamino, Carboxy und/oder Alkoxycarbonyl substituiertes Phenyl oder Naphthyl, oder für eine gegebenenfalls durch Halogen und/oder Alkyl substituierte heterocyclische Gruppierung aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl oder Pyrimidinyl steht,
$Z^2$ für Wasserstoff, für gegebenenfalls durch Halogen, Alkoxy oder Alkoxycarbonyl substituiertes Alkyl, für gegebenenfalls durch Halogen und/oder Alkyl substituiertes Cycloalkyl, für Alkenyl, Benzyl oder für gegebenenfalls durch Halogen, Cyano, Nitro, Alkyl, Halogenalkyl, Alkoxy, Halogenalkoxy, Alkylthio, Halogenalkylthio und/oder Alkoxycarbonyl substituiertes Phenyl, für Alkoxy, Benzyloxy, Phenoxy, Alkylthio, Benzylthio, Phenylthio oder Dialkylamino steht, und
$Z^3$ und $Z^4$ jeweils für gegebenenfalls durch Halogen substituiertes Alkyl oder Alkoxy stehen,
gefunden.

Weiter wurde gefunden, daß man die neuen halogensubstituierten Phenoxybenzyl(thi)ol-Derivate der allgemeinen Formel (I) erhält, wenn man

(a) halogensubstituierte Phenoxybenzylhalogenide der allgemeinen Formel (II)

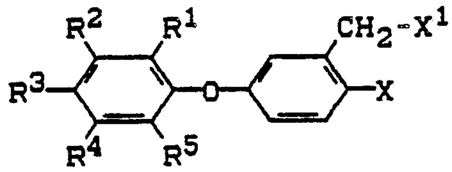

(II)

in welcher
$R^1$, $R^2$, $R^3$, $R^4$, $R^5$ und X die oben angegebenen Bedeutungen haben und
$X^1$ für Halogen steht,
mit nucleophilen Verbindungen der allgemeinen Formel (III)
M - Y - Z (III)
in welcher
Y und Z die oben angegebenen Bedeutungen haben und
M für Wasserstoff, Natrium oder Kalium steht,
gegebenenfalls in Gegenwart von Säureakzeptoren und gegebenenfalls in Gegenwart von Verdünnungsmitteln umsetzt, oder wenn man

(b) halogensubstituierte Phenoxybenzyl(thi)ole der allgemeinen Formel (Ia)

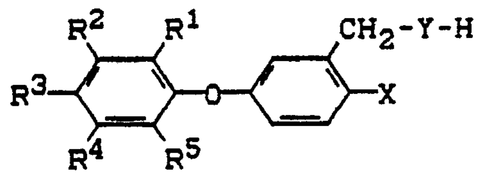

(Ia)

in welcher
$R^1$, $R^2$, $R^3$, $R^4$, $R^5$ X und Y die oben angegebenen Bedeutungen haben,
mit elektrophilen Verbindungen der allgemeinen Formel (IV)
$X^2$ - Z (IV)
in welcher
Z die oben angegebene Bedeutung hat und
$X^2$ für eine nucleophile Abgangsgruppe steht,
gegebenenfalls in Gegenwart von Säureakzeptoren und gegebenenfalls in Gegenwart von Verdünnungsmitteln umsetzt, oder wenn man

(c) halogensubstituierte Phenoxybenzylthiol-Derivate der allgemeinen Formel (Ib)

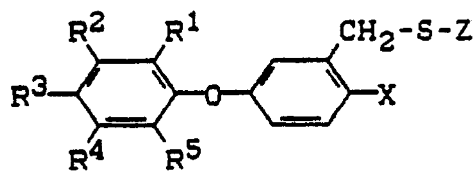

(Ib)

in welcher
$R^1$, $R^2$, $R^3$, $R^4$, $R^5$, X und Z die oben angegebenen Bedeutungen haben,
mit Oxidationsmitteln, gegebenenfalls in Gegenwart von Verdünnungsmitteln umsetzt.

Schließlich wurde gefunden, daß die neuen halogensubstituierten Phenoxybenzyl(thi)ol-Derivate der Formel (I) hervorragende herbizide Eigenschaften aufweisen.

Überraschenderweise sind die erfindungsgemäßen halogensubstituierten Phenoxybenzyl(thi)ol-Derivate der Formel (I) gegen Unkräuter wesentlich stärker wirksam als 3-(2,4-Dichlor-phenoxy)-6-nitro-benzoesäuremethylester, welches ein strukturell ähnlicher vorbekannter Wirkstoff gleicher Wirkungsrichtung ist.

Die Erfindung betrifft vorzugsweise Verbindungen der Formel (I), in welcher
$R^1$ für Wasserstoff, Fluor, Chlor, Brom, Cyano oder Trifluormethyl steht,
$R^2$ für Wasserstoff, Fluor, Chlor oder Brom steht,
$R^3$ für Fluor, Chlor, Brom, Cyano, Trifluormethyl, Trifluormethoxy oder Trifluormethylsulfonyl steht,
$R^4$ für Wasserstoff, Fluor, Chlor oder Brom steht,

$R^5$ für Wasserstoff, Fluor, Chlor oder Brom steht

- mit der Maßgabe, daß wenigstens zwei der Substituenten $R^1$, $R^2$, $R^4$ und $R^5$ von Wasserstoff verschieden sind -

X für Fluor, Chlor oder Brom steht,

Y für Sauerstoff, Schwefel, Sulfinyl oder Sulfonyl steht und

Z für Wasserstoff, für gegebenenfalls durch Cyano, Fluor, Chlor, Brom, Hydroxy, $C_1$-$C_6$-Alkoxy, $C_1$-$C_6$-Alkylthio, Carboxy, $C_1$-$C_6$-Alkoxy-carbonyl, $C_1$-$C_6$-Alkyl-carbonyloxy oder $C_1$-$C_6$-Alkoxy-carbonyl-$C_1$-$C_6$-alkoxy-carbonyl substituiertes $C_1$-$C_6$-Alkyl, für jeweils gegebenenfalls durch Fluor, Chlor, Brom und/oder $C_1$-$C_4$-Alkyl substituiertes $C_3$-$C_8$-Cycloalkyl oder $C_3$-$C_8$-Cycloalkyl-$C_1$-$C_4$-alkyl, für jeweils gegebenenfalls durch Fluor, Chlor und/oder Brom substituiertes $C_3$-$C_6$-Alkenyl oder $C_3$-$C_6$-Alkinyl, oder für eine der nachstehenden Gruppierungen steht

$-(CH_2)_m-Z^1$, 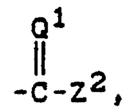, 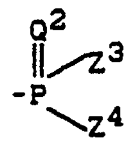

worin

m für die Zahlen 0, 1, 2 oder 3 steht,

$Q^1$ und $Q^2$ jeweils für Sauerstoff oder Schwefel stehen,

$Z^1$ für jeweils gegebenenfalls durch Fluor, Chlor, Brom, Iod, Cyano, Nitro, $C_1$-$C_4$-Alkyl, fluorsubstituiertes und/oder chlorsubstituiertes $C_1$-$C_2$-Alkyl, $C_1$-$C_4$-Alkoxy, fluorsubstituiertes und/oder chlorsubstituiertes $C_1$-$C_2$-Alkoxy, $C_1$-$C_4$-Alkylthio, fluorsubstituiertes und/oder chlorsubstituiertes $C_1$-$C_2$-Alkylthio, Di-($C_1$-$C_2$-alkyl)-amino, Carboxy und/oder $C_1$-$C_4$-Alkoxy-carbonyl substituiertes Phenyl oder Naphthyl, oder für eine gegebenenfalls durch Fluor, Chlor, Brom und/oder $C_1$-$C_4$-Alkyl substituierte heterocyclische Gruppierung aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl oder Pyrimidinyl steht,

$Z^2$ für Wasserstoff, für gegebenenfalls durch Fluor, Chlor, Brom, $C_1$-$C_4$-Alkoxy oder $C_1$-$C_4$-Alkoxy-carbonyl substituiertes $C_1$-$C_6$-Alkyl, für gegebenenfalls durch Fluor, Chlor, Brom und/ oder $C_1$-$C_4$-Alkyl substituiertes $C_3$-$C_6$-Cycloalkyl, für $C_3$-$C_6$-Alkenyl, Benzyl oder für gegebenenfalls durch Fluor, Chlor, Brom, Iod, Cyano, Nitro, $C_1$-$C_4$-Alkyl, fluorsubstituiertes und/oder chlorsubstituiertes $C_1$-$C_2$-Alkyl, $C_1$-$C_4$-Alkoxy, fluorsubstituiertes und/oder chlorsubstituiertes $C_1$-$C_2$-Alkoxy, $C_1$-$C_4$-Alkylthio, fluorsubstituiertes und/oder chlorsubstituiertes $C_1$-$C_2$-Alkylthio und/oder $C_1$-$C_4$-Alkoxy-carbonyl substituiertes Phenyl, für $C_1$-$C_4$-Alkoxy, Benzyloxy, Phenoxy, $C_1$-$C_4$-Alkylthio, Benzylthio, Phenylthio oder Di-($C_1$-$C_4$-alkyl)-amino steht und

$Z^3$ und $Z^4$ jeweils für gegebenenfalls durch Fluor und/oder Chlor substituiertes $C_1$-$C_4$-Alkyl oder $C_1$-$C_4$-Alkoxy stehen.

Die Erfindung betrifft insbesondere Verbindungen der Formel (I), in welcher

$R^1$ für Chlor steht,

$R^2$ für Wasserstoff steht,

$R^3$ für Trifluormethyl steht,

$R^4$ für Wasserstoff, Fluor oder Chlor steht,

$R^5$ für Fluor oder Chlor steht,

X für Chlor oder Brom steht,

Y für Sauerstoff, Schwefel, Sulfinyl oder Sulfonyl steht und

Z für Wasserstoff, für gegebenenfalls durch Cyano, Fluor, Chlor, Hydroxy, $C_1$-$C_4$-Alkoxy, $C_1$-$C_4$-Alkylthio, Carboxy, $C_1$-$C_4$-Alkoxy-carbonyl, $C_1$-$C_4$-Alkylcarbonyloxy oder $C_1$-$C_4$-Alkoxy-carbonyl-$C_1$-$C_2$-alkoxy-carbonyl substituiertes $C_1$-$C_4$-Alkyl, für jeweils gegebenenfalls durch Chlor und/oder Methyl substituiertes $C_3$-$C_6$-Cycloalkyl oder $C_3$-$C_6$-Cycloalkyl-$C_1$-$C_2$-alkyl, für jeweils gegebenenfalls durch Chlor substituiertes $C_3$-$C_4$-Alkenyl oder $C_3$-$C_4$-Alkinyl, oder für eine der nachstehenden Gruppierungen steht

$-(CH_2)_m-Z^1$, 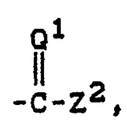, 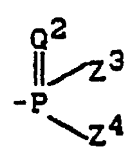

worin
m für die Zahlen 0, 1 oder 2 steht,
$Q^1$ und $Q^2$ jeweils für Sauerstoff oder Schwefel stehen,
$Z^1$ für jeweils gegebenenfalls durch Fluor, Chlor, Brom, Cyano, Nitro, Methyl, Ethyl, Trifluormethyl, Methoxy, Ethoxy, Difluormethoxy, Trifluormethoxy, Methylthio, Ethylthio, Difluormethylthio, Trifluormethylthio, Dimethylamino und/oder Methoxycarbonyl substituiertes Phenyl oder Naphthyl, oder für eine gegebenenfalls durch Chlor und/oder Methyl substituierte heterocyclische Gruppierung aus der Reihe Furyl, Tetrahydrofuryl, Thienyl, Perhydropyranyl, Oxazolyl, Thiazolyl oder Dioxolanyl steht,
$Z^2$ für Wasserstoff, für gegebenenfalls durch Fluor, Chlor, $C_1$-$C_2$-Alkoxy oder $C_1$-$C_2$-Alkoxycarbonyl substituiertes $C_1$-$C_4$-Alkyl, für gegebenenfalls durch Chlor und/oder Methyl substituiertes $C_3$-$C_6$-Cycloalkyl, für $C_3$-$C_4$-Alkenyl, Benzyl, für gegebenenfalls durch Fluor, Chlor, Brom, Nitro, Methyl, Ethyl, Trifluormethyl, Methoxy, Ethoxy, Difluormethoxy, Trifluormethoxy und/oder Methoxycarbonyl substituiertes Phenyl oder für Dimethylamino steht, und
$Z^3$ und $Z^4$ jeweils für $C_1$-$C_3$-Alkoxy stehen.

Beispiele für die erfindungsgemäßen Verbindungen der Formel (I) sind in der nachstehenden Tablle 1 aufgeführt - vgl. auch die Herstellungsbeispiele.

**Tabelle 1: Beispiele für die Verbindungen der Formel (I)**

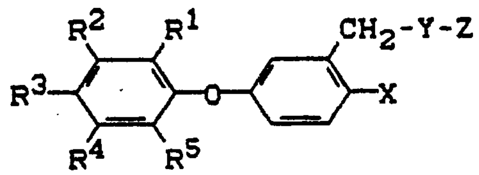

(I)

| $R^1$ | $R^2$ | $R^3$ | $R^4$ | $R^5$ | X | Y | Z |
|---|---|---|---|---|---|---|---|
| Cl | H | $CF_3$ | H | Cl | Cl | O | H |
| Cl | H | $CF_3$ | H | Cl | Cl | S | H |
| Cl | H | $CF_3$ | H | F | Cl | O | H |
| Cl | H | $CF_3$ | H | F | Cl | S | H |
| Cl | H | $CF_3$ | Cl | Cl | Cl | O | H |
| Cl | H | $CF_3$ | Cl | Cl | Cl | S | H |
| Cl | H | $CF_3$ | F | Cl | Cl | O | H |

Tabelle 1 - Fortsetzung

| $R^1$ | $R^2$ | $R^3$ | $R^4$ | $R^5$ | X | Y | Z |
|---|---|---|---|---|---|---|---|
| Cl | H | $CF_3$ | F | Cl | Cl | S | H |
| Cl | H | $CF_3$ | H | Cl | Br | O | H |
| Cl | H | $CF_3$ | H | Cl | Br | S | H |
| Cl | H | $CF_3$ | H | F | Br | O | H |
| Cl | H | $CF_3$ | H | F | Br | S | H |
| Cl | H | $CF_3$ | Cl | Cl | Br | O | H |
| Cl | H | $CF_3$ | Cl | Cl | Br | S | H |
| Cl | H | $CF_3$ | F | Cl | Br | O | H |
| Cl | H | $CF_3$ | F | Cl | Br | S | H |
| Cl | H | $CF_3$ | H | Cl | Cl | O | $CH_3$ |
| Cl | H | $CF_3$ | H | Cl | Cl | S | $CH_3$ |
| Cl | H | $CF_3$ | H | Cl | Cl | O | $CHF_2$ |
| Cl | H | $CF_3$ | H | Cl | Cl | S | $CHF_2$ |
| Cl | H | $CF_3$ | H | Cl | Cl | O | $CF_2CHFCl$ |
| Cl | H | $CF_3$ | H | Cl | Cl | S | $CF_2CHFCl$ |
| Cl | H | $CF_3$ | H | Cl | Cl | O | $C_2H_5$ |
| Cl | H | $CF_3$ | H | Cl | Cl | S | $C_2H_5$ |
| Cl | H | $CF_3$ | H | Cl | Cl | O | $C_3H_7$ |
| Cl | H | $CF_3$ | H | Cl | Cl | S | $C_3H_7$ |
| Cl | H | $CF_3$ | H | Cl | Cl | O | $CH(CH_3)_2$ |

Tabelle 1 - Fortsetzung

| $R^1$ | $R^2$ | $R^3$ | $R^4$ | $R^5$ | X | Y | Z |
|---|---|---|---|---|---|---|---|
| Cl | H | $CF_3$ | H | Cl | Cl | S | $CH(CH_3)_2$ |
| Cl | H | $CF_3$ | H | Cl | Cl | O | $C_4H_9$ |
| Cl | H | $CF_3$ | H | Cl | Cl | S | $CH_2CH(CH_3)_2$ |
| Cl | H | $CF_3$ | H | Cl | Cl | O | $CH_2CH_2OH$ |
| Cl | H | $CF_3$ | H | Cl | Cl | S | $CH_2CH_2OH$ |
| Cl | H | $CF_3$ | H | Cl | Cl | O | $CH_2CH_2CN$ |
| Cl | H | $CF_3$ | H | Cl | Cl | S | $CH_2CH_2CN$ |
| Cl | H | $CF_3$ | H | Cl | Cl | O | $CH_2CN$ |
| Cl | H | $CF_3$ | H | Cl | Cl | S | $CH_2CN$ |
| Cl | H | $CF_3$ | H | Cl | Cl | O | $CH_2CH_2OCH_3$ |
| Cl | H | $CF_3$ | H | Cl | Cl | S | $CH_2CH_2OCH_3$ |
| Cl | H | $CF_3$ | H | Cl | Cl | O | $CH_2CH_2SCH_3$ |
| Cl | H | $CF_3$ | H | Cl | Cl | S | $CH_2CH_2SCH_3$ |
| Cl | H | $CF_3$ | H | Cl | Cl | O | $CH_2COOH$ |
| Cl | H | $CF_3$ | H | Cl | Cl | S | $CH_2COOH$ |
| Cl | H | $CF_3$ | H | Cl | Cl | O | $CH_2COOCH_3$ |
| Cl | H | $CF_3$ | H | Cl | Cl | S | $CH_2COOCH_3$ |
| Cl | H | $CF_3$ | H | Cl | Cl | O | $CH_2COOC_2H_5$ |
| Cl | H | $CF_3$ | H | Cl | Cl | S | $CH_2COOC_2H_5$ |
| Cl | H | $CF_3$ | H | Cl | Cl | O | $CH_2COOCH_2COOCH_3$ |

<u>Tabelle 1</u> - Fortsetzung

| $R^1$ | $R^2$ | $R^3$ | $R^4$ | $R^5$ | X | Y | Z |
|---|---|---|---|---|---|---|---|
| Cl | H | $CF_3$ | H | Cl | Cl | S | $CH_2COOCH_2COOCH_3$ |
| Cl | H | $CF_3$ | H | Cl | Cl | O | $CH_2COOCHCOOCH_3$<br>\|<br>$CH_3$ |
| Cl | H | $CF_3$ | H | Cl | Cl | S | $CH_2COOCHCOOCH_3$<br>\|<br>$CH_3$ |
| Cl | H | $CF_3$ | H | Cl | Cl | O | $CHCOOCH_3$<br>\|<br>$CH_3$ |
| Cl | H | $CF_3$ | H | Cl | Cl | S | $CHCOOCH_3$<br>\|<br>$CH_3$ |
| Cl | H | $CF_3$ | H | Cl | Cl | O | $CHCOOC_2H_5$<br>\|<br>$CH_3$ |
| Cl | H | $CF_3$ | H | Cl | Cl | S | $CHCOOC_2H_5$<br>\|<br>$CH_3$ |
| Cl | H | $CF_3$ | H | Cl | Cl | O | 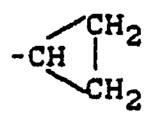 |
| Cl | H | $CF_3$ | H | Cl | Cl | S | 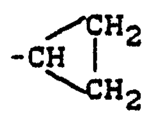 |
| Cl | H | $CF_3$ | H | Cl | Cl | O | 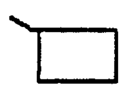 |
| Cl | H | $CF_3$ | H | Cl | Cl | S | 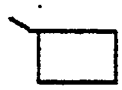 |

**Tabelle 1** - Fortsetzung

| $R^1$ | $R^2$ | $R^3$ | $R^4$ | $R^5$ | X | Y | Z |
|---|---|---|---|---|---|---|---|
| Cl | H | $CF_3$ | H | Cl | Cl | O | 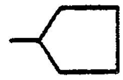 |
| Cl | H | $CF_3$ | H | Cl | Cl | S | 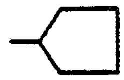 |
| Cl | H | $CF_3$ | H | Cl | Cl | O | 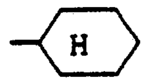 |
| Cl | H | $CF_3$ | H | Cl | Cl | S | 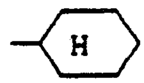 |
| Cl | H | $CF_3$ | H | Cl | Cl | S | 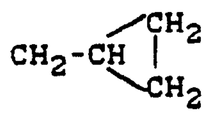 |
| Cl | H | $CF_3$ | H | Cl | Cl | S | 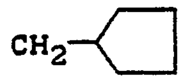 |
| Cl | H | $CF_3$ | H | Cl | Cl | S | 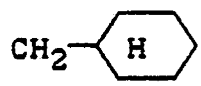 |
| Cl | H | $CF_3$ | H | Cl | Cl | O | $CH_2CH{=}CH_2$ |
| Cl | H | $CF_3$ | H | Cl | Cl | O | $CH_2CH{=}CHCH_3$ |
| Cl | H | $CF_3$ | H | Cl | Cl | S | $CH_2CH{=}CH_2$ |
| Cl | H | $CF_3$ | H | Cl | Cl | O | $CH_2C{\equiv}CH$ |
| Cl | H | $CF_3$ | H | Cl | Cl | S | $CH_2C{\equiv}CH$ |
| Cl | H | $CF_3$ | H | Cl | Cl | O | 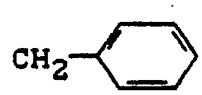 |

Tabelle 1 - Fortsetzung

| $R^1$ | $R^2$ | $R^3$ | $R^4$ | $R^5$ | X | Y | Z |
|---|---|---|---|---|---|---|---|
| Cl | H | $CF_3$ | H | Cl | Cl | S | 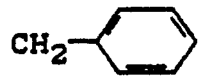 |
| Cl | H | $CF_3$ | H | Cl | Cl | S | 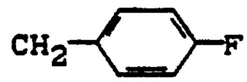 |
| Cl | H | $CF_3$ | H | Cl | Cl | S | 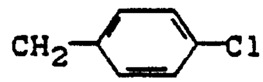 |
| Cl | H | $CF_3$ | H | Cl | Cl | O | 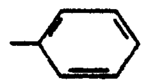 |
| Cl | H | $CF_3$ | H | Cl | Cl | S | 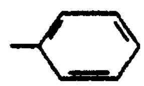 |
| Cl | H | $CF_3$ | H | Cl | Cl | O | 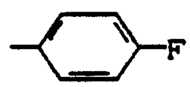 |
| Cl | H | $CF_3$ | H | Cl | Cl | O | 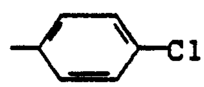 |
| Cl | H | $CF_3$ | H | Cl | Cl | O | 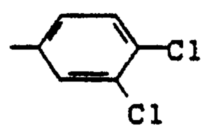 |
| Cl | H | $CF_3$ | H | Cl | Cl | O | 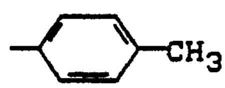 |
| Cl | H | $CF_3$ | H | Cl | Cl | O | 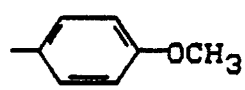 |

**Tabelle 1** - Fortsetzung

| $R^1$ | $R^2$ | $R^3$ | $R^4$ | $R^5$ | X | Y | Z |
|---|---|---|---|---|---|---|---|
| Cl | H | $CF_3$ | H | Cl | Cl | O | 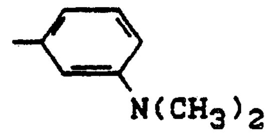 |
| Cl | H | $CF_3$ | H | Cl | Cl | O | 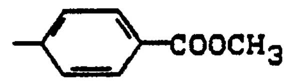 |
| Cl | H | $CF_3$ | H | Cl | Cl | O | 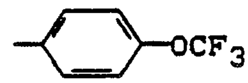 |
| Cl | H | $CF_3$ | H | Cl | Cl | O | 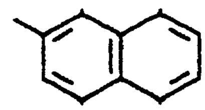 |
| Cl | H | $CF_3$ | H | Cl | Cl | O | 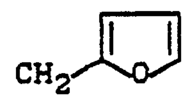 |
| Cl | H | $CF_3$ | H | Cl | Cl | O | 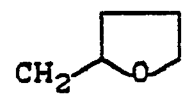 |
| Cl | H | $CF_3$ | H | Cl | Cl | O | 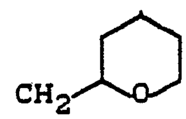 |
| Cl | H | $CF_3$ | H | Cl | Cl | O | $CO-CH_3$ |
| Cl | H | $CF_3$ | H | Cl | Cl | O | $CO-C_2H_5$ |
| Cl | H | $CF_3$ | H | Cl | Cl | O | $CO-C_3H_7$ |
| Cl | H | $CF_3$ | H | Cl | Cl | O | $CO-CH(CH_3)_2$ |
| Cl | H | $CF_3$ | H | Cl | Cl | O | $CO-C_4H_9$ |
| Cl | H | $CF_3$ | H | Cl | Cl | O | $CF_3$ |
| Cl | H | $CF_3$ | H | Cl | Cl | O | $CO-CH_2Cl$ |

**Tabelle 1** - Fortsetzung

| $R^1$ | $R^2$ | $R^3$ | $R^4$ | $R^5$ | X | Y | Z |
|---|---|---|---|---|---|---|---|
| Cl | H | $CF_3$ | H | Cl | Cl | O | $CO-CHCl_2$ |
| Cl | H | $CF_3$ | H | Cl | Cl | O | $CO-CCl_3$ |
| Cl | H | $CF_3$ | H | Cl | Cl | O | $CO-CH_2CH_2Cl$ |
| Cl | H | $CF_3$ | H | Cl | Cl | O | $CO-CH_2OCH_3$ |
| Cl | H | $CF_3$ | H | Cl | Cl | O | $CO-CH_2COOCH_3$ |
| Cl | H | $CF_3$ | H | Cl | Cl | O | $CO-CH_2COOC_2H_5$ |
| Cl | H | $CF_3$ | H | Cl | Cl | O | 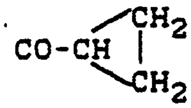 |
| Cl | H | $CF_3$ | H | Cl | Cl | O | 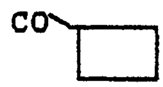 |
| Cl | H | $CF_3$ | H | Cl | Cl | O | 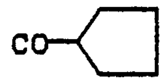 |
| Cl | H | $CF_3$ | H | Cl | Cl | O | 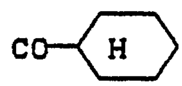 |
| Cl | H | $CF_3$ | H | Cl | Cl | O | $CO-CH=CHCH_3$ |
| Cl | H | $CF_3$ | H | Cl | Cl | O | 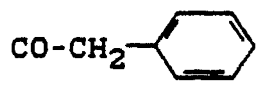 |
| Cl | H | $CF_3$ | H | Cl | Cl | O | 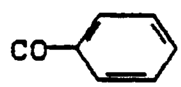 |
| Cl | H | $CF_3$ | H | Cl | Cl | O | 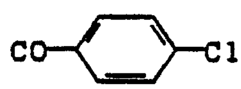 |

Tabelle 1 - Fortsetzung

| $R^1$ | $R^2$ | $R^3$ | $R^4$ | $R^5$ | X | Y | Z |
|---|---|---|---|---|---|---|---|
| Cl | H | $CF_3$ | H | Cl | Cl | O | 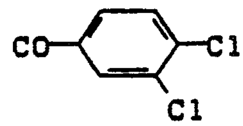 |
| Cl | H | $CF_3$ | H | Cl | Cl | O | 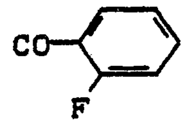 |
| Cl | H | $CF_3$ | H | Cl | Cl | O | 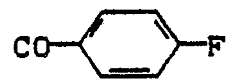 |
| Cl | H | $CF_3$ | H | Cl | Cl | O | 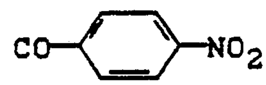 |
| Cl | H | $CF_3$ | H | Cl | Cl | O | 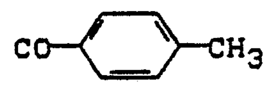 |
| Cl | H | $CF_3$ | H | Cl | Cl | O | 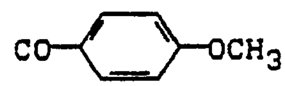 |
| Cl | H | $CF_3$ | H | Cl | Cl | O | 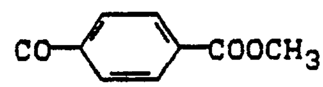 |
| Cl | H | $CF_3$ | H | Cl | Cl | O | $\overset{O}{\overset{\|}{P}}(OCH_3)_2$ |
| Cl | H | $CF_3$ | H | Cl | Cl | O | $\overset{O}{\overset{\|}{P}}(OC_2H_5)_2$ |
| Cl | H | $CF_3$ | H | Cl | Cl | S | $\overset{O}{\overset{\|}{P}}(OCH_3)_2$ |

Tabelle 1 - Fortsetzung

| $R^1$ | $R^2$ | $R^3$ | $R^4$ | $R^5$ | X | Y | Z |
|---|---|---|---|---|---|---|---|
| Cl | H | $CF_3$ | H | Cl | Cl | S | O<br>‖<br>$P(OC_2H_5)_2$ |
| Cl | H | $CF_3$ | H | Cl | Cl | O | S<br>‖<br>$P(OC_2H_5)_2$ |
| Cl | H | $CF_3$ | H | Cl | Cl | S | S<br>‖<br>$P(OC_2H_5)_2$ |
| Cl | H | $CF_3$ | H | F | Cl | O | $CH_2COOCH_3$ |
| Cl | H | $CF_3$ | H | F | Cl | S | $CH_2COOCH_3$ |
| Cl | H | $CF_3$ | H | F | Cl | O | $CH_2COOC_2H_5$ |
| Cl | H | $CF_3$ | H | F | Cl | S | $CH_2COOC_2H_5$ |
| Cl | H | $CF_3$ | H | F | Cl | S | $CH_2COOCH_2COOCH_3$ |
| Cl | H | $CF_3$ | H | F | Cl | S | $CH_2COOCH_2COOC_2H_5$ |
| Cl | H | $CF_3$ | H | F | Cl | S | $CH_2COOCHCOOCH_3$<br>\|<br>$CH_3$ |
| Cl | H | $CF_3$ | H | F | Cl | S | $CH_2COOCHCOOC_2H_5$<br>\|<br>$CH_3$ |
| Cl | H | $CF_3$ | H | F | Cl | O | $CO-CH_2COOCH_3$ |
| Cl | H | $CF_3$ | H | F | Cl | O | $CO-CH_2COOC_2H_5$ |

Verwendet man für das erfindungsgemäße Verfahren (a) 2-Fluor-5-(2,6-difluor-4-trifluormethyl-phenoxy)-benzylbromid und 2-Ethylthio-ethanol als Ausgangsstoffe, so kann der Reaktionsablauf durch das folgende Formelschema wiedergegeben werden:

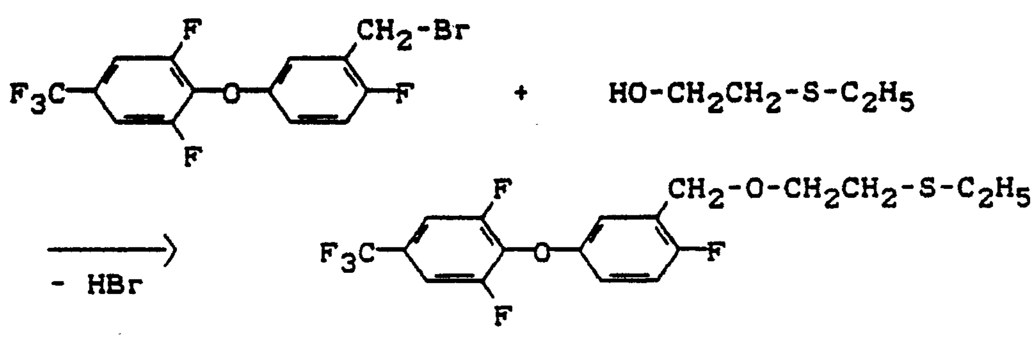

Verwendet man für das erfindungsgemäße Verfahren (b) 2-Brom-5-(2-chlor-5,6-difluor-4-trifluormethyl-phenoxy)benzylthiol und Chloracetonitril als Ausgangsstoffe, so kann der Reaktionsablauf durch das folgende Formelschema wiedergegeben werden:

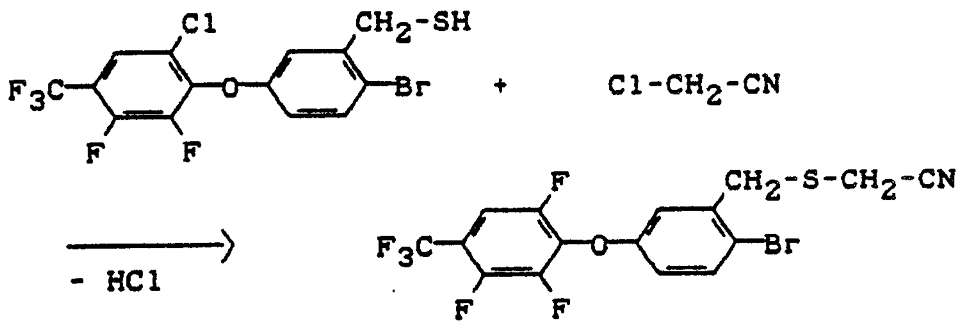


Verwendet man für das erfindungsgemäße Verfahren (c) 5-(2-Chlor-5-(2,6-dichlor-4-trifluormethyl-phenoxy)-benzyl)-mercaptoessigsäure-methylester und Hydrogenperoxid als Ausgangsstoffe, so kann der Reaktionsablauf durch das folgende Formelschema wiedergegeben werden:

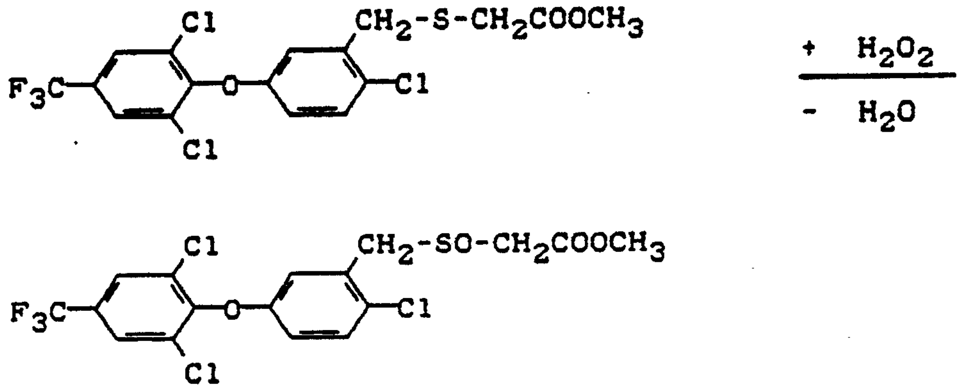


Die beim erfindungsgemäßen Verfahren (a) zur Herstellung von Verbindungen der Formel (I) als Ausgangsstoffe zu verwendenden halogensubstituierten Phenoxybenzylhalogenide sind durch die Formel (II) allgemein definiert.

In Formel (II) haben $R^1$, $R^2$, $R^3$, $R^4$, $R^5$ und X vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$, $R^3$, $R^4$, $R^5$ und X angegeben wurden und $X^1$ steht vorzugsweise für Chlor oder Brom.

Beispiele für die Ausgangsstoffe der Formel (II) sind in der nachstehenden Tabelle 2 aufgeführt.

**Tabelle 2:** Beispiele für die Ausgangsstoffe der Formel (II)

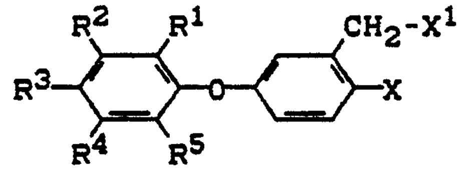

(II)

| $R^1$ | $R^2$ | $R^3$ | $R^4$ | $R^5$ | X | $X^1$ |
|---|---|---|---|---|---|---|
| Cl | H | $CF_3$ | H | Cl | Cl | Cl |
| Cl | H | $CF_3$ | H | Cl | Br | Cl |
| Cl | H | $CF_3$ | H | Cl | Cl | Br |
| Cl | H | $CF_3$ | H | Cl | Br | Br |
| Cl | H | $CF_3$ | H | F | Cl | Cl |
| Cl | H | $CF_3$ | H | F | Br | Cl |
| Cl | H | $CF_3$ | H | F | Cl | Br |
| Cl | H | $CF_3$ | H | F | Br | Br |
| Cl | H | $CF_3$ | F | Cl | Cl | Cl |
| Cl | H | $CF_3$ | F | Cl | Br | Cl |
| Cl | H | $CF_3$ | F | Cl | Cl | Br |
| Cl | H | $CF_3$ | F | Cl | Br | Br |
| Cl | H | $CF_3$ | Cl | Cl | Cl | Cl |
| Cl | H | $CF_3$ | Cl | Cl | Br | Cl |
| Cl | H | $CF_3$ | Cl | Cl | Cl | Br |
| Cl | H | $CF_3$ | Cl | Cl | Br | Br |
| Cl | H | $CF_3$ | F | F | Cl | Cl |

Die Ausgangsstoffe der Formel (II) sind bekannt (vgl. EP-A 23 890, EP-A 56 119) bzw. Gegenstand einer nicht vorveröffentlichten Patentanmeldung [P 38 12 768.7 vom 16.4.1988 (Le A 25 995)].

Man erhält die Ausgangsverbindungen der Formel (II), wenn man entsprechende halogensubstituierte Phenoxytoluol-Derivate der allgemeinen Formel (V)

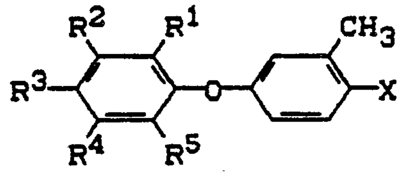

(V)

in welcher
$R^1$, $R^2$, $R^3$, $R^4$, $R^5$ und X die oben angegebenen Bedeutungen haben, mit Halogenierungsmitteln, wie z. B. Chlor, Brom, Sulfurylchlorid, N-Chlor- oder N-Brom-succinimid, gegebenenfalls unter Belichtung, beispielsweise mit Quecksilbertauchlampen, gegebenenfalls in Gegenwart von Katalysatoren, wie z. B. Benzoylperoxid oder Azo-bis-isobutyronitril, und gegebenenfalls in Gegenwart von Verdünnungmitteln, wie z. B. Tetrachlormethan, bei Temperaturen zwischen 20 °C und 150 °C umsetzt.

Die halogensubstituierten Phenoxytoluol-Derivate der Formel (V) sind bekannt und/oder können nach an sich bekannten Verfahren hergestellt werden (vgl. DE-AS 23 04 006).

Die beim erfindungsgemäßen Verfahren (a) weiter als Ausgangsstoffe zu verwendenden nucleophilen Verbindungen sind durch die Formel (III) allgemein definiert.

In Formel (III) haben Y und Z vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für Y und Z angegeben wurden und M steht vorzugsweise für Wasserstoff oder Natrium.

Als Beispiele für die Ausgangsstoffe der Formel (III) seien genannt:
Wasser, Schwefelwasserstoff, Methanol, Ethanol, Propanol, Isopropanol, Butanol, Isobutanol, sec-Butanol, tert-Butanol, Methanthiol, Ethanthiol, Propanthiol, 1-Methyl-ethanthiol, Butanthiol, 2-Methyl-butanthiol, und 1,1-Dimethyl-ethanthiol, Ethylenglycol, Propylenglycol, 2-Methoxy- und 2-Ethoxy-ethanol, 2-Methylthio- und 2-Ethylthio-ethanol, Glycolsäure und Milchsäure sowie deren Methylester und Ethylester, Cyclopropyl-, Cyclobutyl-, Cyclopentyl- und Cyclohexylalkohol, Cyclopropyl-, Cyclobutyl-, Cyclopentyl- und Cyclohexylmethanol, Allylalkohol, Crotylalkohol, Propargylalkohol, Benzylalkohol, Phenol, 2-Fluor-, 4-Fluor-, 2-Chlor-, 3-Chlor-, 4-Chlor-, 3,4-Dichlor-, 4-Brom-, 4-Iod-, 2-Methyl-, 3-Methyl-, 4-Methyl-, 3,4-Dimethyl-, 2-Methoxy-, 3-Methoxy-, 4-Methoxy-, 3,4-Dimethoxy-, 3-Dimethylamino-, 4-Dimethylamino-, 2-Methoxycarbonyl-, 3-Methoxycarbonyl-, 4-Methoxycarbonyl-, 3-Trifluormethoxy- und 4-Trifluormethoxy-phenol, 1-Naphthol, 2-Naphthol, 2-Hydroxymethyl furan, 2-Hydroxymethyl-tetrahydrofuran, 2-Hydroxymethylperhydropyran und 4-Hydroxymethyl-1,3-dioxolan.

Die Ausgangsstoffe der Formel (III) sind bekannte organische Synthesechemikalien.

Das erfindungsgemäße Verfahren (a) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid Tetramethylensulfon und Hexamethylphosphorsäuretriamid.

Als Säureakzeptoren können bei dem erfindungsgemäßen Verfahren (a) alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate, -hydride und -alkoholate wie Natrium- und Kaliumcarbonat, Natrium-und Kalium-hydrid, Natrium- und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (a) in einem größeren Bereich variiert werden. Im allgemeinen arbeitet man bei Temperaturen zwischen 0 °C und 150 °C, vorzugsweise bei Temperaturen zwischen 10 °C und 100 °C.

Das erfindungsgemäße Verfahren (a) wird im allgemeinen bei Normaldruck durchgeführt.

Zur Durchführung des erfindungsgemäßen Verfahrens (a) werden die jeweils benötigten Ausgangsstoffe im allgemeinen in angenähert äquimolaren Mengen eingesetzt. Es ist jedoch auch möglich, eine der beiden

jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (a) jeweils nach üblichen Methoden.

Die beim erfindungsgemäßen Verfahren (b) zur Herstellung von Verbindungen der Formel (I) als Ausgangsstoffe zu verwendenden halogensubstituierten Phenoxybenzyl(thi)ole sind durch die Formel (Ia) allgemein definiert.

In Formel (Ia) haben $R^1$, $R^2$, $R^3$, $R^4$, $R^5$, X und Y vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$, $R^3$, $R^4$, $R^5$, X und Y angegeben wurden.

Beispiele für die Ausgangsstoffe der Formel (Ia) sind Tabelle 1 (Z = H) zu entnehmen.

Die Ausgangsstoffe der Formel (Ia) sind erfindungsgemäße, neue Verbindungen; sie können nach dem erfindungsgemäßen Verfahren (a) hergestellt werden.

Die beim erfindungsgemäßen Verfahren (b) weiter als Ausgangsstoffe zu verwendenden elektrophilen Verbindungen sind durch die Formel (IV) allgemein definiert.

In Formel (IV) hat Z vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für Z angegeben wurde und $X^2$ steht vorzugsweise für Chlor, Brom, Iod, gegebenenfalls durch Fluor oder Chlor substituiertes $C_1$-$C_4$-Alkylsulfonyloxy oder gegebenenfalls durch Fluor, Chlor, Brom oder Methyl substituier tes Phenylsulfonyloxy, insbesondere für Chlor, Brom, Methylsulfonyloxy, Phenylsulfonyloxy oder 4-Methylphenylsulfonyloxy.

Als Beispiele für die Ausgangsstoffe der Formel (IV) seien genannt:

Methyl-, Ethyl-, Propyl-, Isopropyl-, Butyl-, Isobutyl-, sec-Butyl- und tert-Butyl-chlorid, - bromid und -iodid, Chlordifluormethan, 1,2-Dichlor-1,1,2,2-tetrafluorethan, Allylchlorid, Benzylchlorid, 2-Fluor-, 4-Fluor-, 2-Chlor-, 3-Chlor-, 4-Chlor-, 2,6-Dichlor-, 4-Methyl-, 4-Methoxy- und 4-Methoxycarbonyl-benzylchlorid, Acetylchlorid, Propionsäurechlorid, Buttersäurechlorid, Isobuttersäurechlorid, Valeriansäurechlorid, Isovaleriansäurechlorid, Chloracetylchlorid, Dichloracetylchlorid, Trichloracetylchlorid, 2-Chlor-propionsäurechlorid, Methoxyacetylchlorid, Cyclopropan-, Cyclobutan-, Cyclopentan- und Cyclohexan-carbonsäurechlorid, Crotonsäurechlorid, Phenylessigsäurechlorid, Benzoylchlorid, 2-Fluor-, 4-Fluor, 2-Chlor-, 3-Chlor-, 4-Chlor-, 3,4-Dichlor-, 2-Nitro-, 4-Nitro-, 4-Methyl-, 4-Methoxy- und 4-Methoxycarbonyl-benzoylchlorid, Phosphorsäurechlorid-dimethylester, -diethylester, -dipropylester, -diisopropylester und -dibutylester sowie Thiophosphorsäure-chlorid-dimethylester, -diethylester, -dipropylester, -diisopropylester und -dibutylester, Chloracetonitril, Bromacetonitril, ß-Brom-propionitril, ß-Chlor-propionitril, α-Chlor-, α-Brom- und α-lod-propionsäure-methylester, -ethylester, -propylester, -isopropylester, -butylester, -isobutylester und -sec-butylester, ß-Chlor-, ß-Brom- und ß-Iod-propionsäure-methylester, -ethylester, -propylester, -isopropylester, -butylester, -isobutylester und -sec-butylester, Chlor-, Brom- und Iod-essigsäure-methylester, -ethylester, -propylester, -isopropylester, -butylester, -isobutylester und -sec-butylester, α-Methylsulfonyloxy-, α-Ethylsulfonyloxy-, α-Propylsulfonyloxy-, α-Butylsulfonyloxy-, α-Trifluormethylsulfonyloxy-, α-Phenylsulfonyloxy- und α-(4-Methylphenylsulfonyloxy)-propionsäure-methylester, -ethylester, -propylester, -butylester, -isopropylester, -isobutylester und -sec-butylester.

Die Ausgangsstoffe der Formel (IV) sind bekannt und/oder können nach an sich bekannten Verfahren hergestellt werden (vgl. DE-OS 27 58 002, DE-OS 28 54 542).

Das erfindungsgemäße Verfahren (b) wird vorzugsweise in Gegenwart von Verdünnungsmitteln durchgeführt. Es kommen vor allem diejenigen organischen Solventien in Betracht, die bereits oben für Verfahren (a) als Verdünnungsmittel angegeben wurden.

Das erfindungsgemäße Verfahren (b) wird gegebenenfalls in Gegenwart von Säurekazeptoren durchgeführt. Es kommen vor allem diejenigen Säurebindemittel in Betracht, die bereits oben für Verfahren (a) angegeben wurden.

Die Reaktionstemperaturen können bei dem erfindungsgemäßen Verfahren (b) in einem größeren Bereich variiert werden. Im allgemeinen arbeitet man bei Temperaturen zwischen 0 °C und 150 °C, vorzugsweise bei Temperaturen zwischen 20 °C und 100 °C.

Verfahren (b) wird im allgemeinen bei Normaldruck durch geführt.

Zur Durchführung des erfindungsgemäßen Verfahrens (b) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die

Aufarbeitung erfolgt bei dem erfindungsgemäßen Verfahren (b) jeweils nach üblichen Methoden.

Die beim erfindungsgemäßen Verfahren (c) zur Herstellung von Verbindungen der Formel (I) als Ausgangsstoffe zu verwendenden halogensubstituierten Phenoxybenzylthiol-Derivate sind durch die Formel (Ib) allgemein definiert.

In Formel (Ib) haben $R^1$, $R^2$, $R^3$, $R^4$, $R^5$, X und Z vorzugsweise bzw. insbesondere diejenigen Bedeutungen, die bereits oben im Zusammenhang mit der Beschreibung der erfindungsgemäßen Verbindungen der Formel (I), vorzugsweise bzw. als insbesondere bevorzugt für $R^1$, $R^2$, $R^3$, $R^4$, $R^5$, X und Z angegeben wurden.

Beispiele für die Ausgangsstoffe der Formel (Ib) sind der Tabelle 1 (Y = S) zu entnehmen.

Die Ausgangsstoffe der Formel (Ib) sind erfindungsgemäße neue Verbindungen; sie können nach den erfindungsgemäßen Verfahren (a) und (b) hergestellt werden.

Das erfindungsgemäße Verfahren (c) wird unter Einsatz eines Oxidationsmittels durchgeführt. Es können die üblichen zur Oxidation von Sulfiden zu Sulfoxiden oder Sulfonen verwendbaren Oxidationsmittel verwendet werden. Hierzu gehören beispielsweise Hydrogenperoxid, Perameisensäure, Peressigsäure, Perbenzoesäure, und 3-Chlorperbenzoesäure.

Das erfindungsgemäße Verfahren (c) wird vorzugsweise unter Verwendung von Verdünnungsmitteln durchgeführt. Hierzu gehören organische Lösungsmittel wie Methylenchlorid, Chloroform, Ameisensäure, Essigsäure und Propionsäure, ferner auch Wasser.

Die Reaktionstemperaturen können bei dem erfindungsgemäßen Verfahren (c) in einem größeren Bereich variiert werden. Im allgemeinen arbeitet man bei Temperaturen zwischen -30 °C und +100 °C, vorzugsweise bei Temperaturen zwischen -10 °C und +80 °C.

Verfahren (c) wird im allgemeinen bei Normaldruck durchgeführt.

Zur Durchführung des erfindungsgemäßen Verfahrens (c) setzt man auf 1 Mol eines halogensubstituierten Phenoxybenzylthiol-Derivates der Formel (Ib) im allgemeinen 0,9 bis 1,5 Moläquivalente, vorzugsweise 1,0 bis 1,2 Moläquivalente eines Oxidationsmittels (zur Herstellung von Sulfoxiden) bzw. 1,9 bis 2,8 Moläquivalente, vorzugsweise 2,0 bis 2,5 Moläquivalente eines Oxidationsmittels (zur Herstellung von Sulfonen) ein.

Die Reaktionskomponenten werden im allgemeinen bei Raumtemperatur oder unter Kühlen zusammengegeben und das Reaktionsgemisch wird bei der jeweils erforderlichen Temperatur bis zum Ende der Umsetzung gerührt. Die Aufarbeitung erfolgt bei dem erfindungsgemäßen Verfahren (c) jeweils nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe eignen sich insbesondere zur selektiven Bekämpfung von dikotylen Unkräutern in monokotylen Kulturen, von allem im Nachauflauf-Verfahren.

Einige der erfindungsgemäßen Verbindungen der Formel (I) zeigen auch fungizide Wirkung, beispielsweise gegen Pyricularia oryzae an Reis.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N′-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage. 2,4-Dichlorphenoxyessigsäure (2,4-D); 4-(2,4-Dichlorphenoxy)-buttersäure (2,4-DB); 2,4-Dichlorphenoxypropionsäure (2,4-DP); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); 2-Chlor-N-{[(4-methoxy-6-methyl-1,3,5-triazin-2-yl)-amino]-carbonyl}-benzolsulfonamid (CHLORSULFURON); N,N-Dimethyl-N′-(3-chlor-4-methylphenyl)-harnstoff (CHLORTOLURON); 2-[4-(2,4-Dichlorphenoxy)-phenoxy]-propionsäure, deren Methyl- oder deren Ethylester (DICLOFOP); 4-Amino-6-t-butyl-3-ethylthio-1,2,4-triazin-5-(4H)-on (ETHIOZIN); 2-{4-[(6-Chlor-2-benzoxazolyl)-oxy]-phenoxy}-propansäure, deren Methyl- oder deren Ethylester (FENOXAPROP); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxybenzonitril (IOXYNIL); N,N-Dimethyl-N′-(4-isopropylphenyl)-harnstoff (ISOPROTURON); (2-Methyl-4-chlorphenoxy)-essigsäure (MCPA); N-Methyl-2-(1,3-benzthiazol-2-yloxy)-acetanilid (MEFENACET); 2-{[[((4-Methoxy-6-methyl-1,3,5-triazin-2-yl)-amino)-carbonyl]-amino]-sulfonyl}-benzoesäure oder deren Methylester (METSULFURON); N-(1-Ethylpropyl)-3,4-dimethyl-2,6-dinitroanilin (PENDIMETHALIN); 0-(6-Chlor-3-phenyl-pyridazin-4-yl)-S-octyl-thiocarbonat (PYRIDATE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON)

und 3,5,6-Trichlor-2-pyridyloxyessigsäure (TRICLOPYR). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

## Herstellungsbeispiele

### Beispiel 1

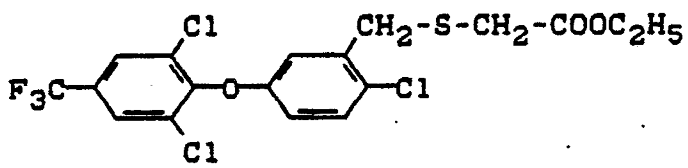

(Verfahren (a))

Eine Mischung aus 2,88 g (0,024 Mol) Mercaptoessigsäureethylester, 0,75 g Natriumhydrid (80 %ig in Paraffinöl) und 150 ml Tetrahydrofuran wird 15 Minuten bei 20 °C gerührt. Dann wird eine Lösung von 8,7 g (0,02 Mol) 2-Chlor-5-(2,6-dichlor-4-trifluormethyl-phenoxy)-benzylbromid in 50 ml Tetrahydrofuran unter Rühren tropfenweise dazu gegeben und anschließend wird die Mischung 2 Stunden bei 60 °C gerührt. Nach Einengen wird der Rückstand in Methylenchlorid/Wasser aufgenommen und ausgeschüttelt. Die organische Phase wird abgetrennt, mit Magnesiumsulfat getrocknet und filtriert. Vom Filtrat wird das Lösungsmittel im Wasserstrahlvakuum sorgfältig abdestilliert.

Man erhält 8,5 g (90 % der Theorie) 2-Chlor-5-(2,6-dichlor-4-trifluormethyl-phenoxy)-benzylthio-essigsäureethylester als hellgelbes Öl.

$^1$H-NMR ($CDCl_3$, $\delta$): 1,15 (t, $OCH_2\underline{CH_3}$); 4,05 (q, $O\underline{CH_2}CH_3$);

8,15 (s, 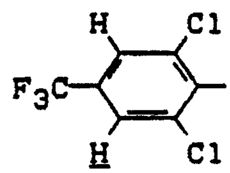 ).

Analog Beispiel 1 sowie nach der allgemeinen Beschreibung der erfindungsgemäßen Herstellungsverfahren können die in der nachstehenden Tabelle 3 aufgeführten Verbindungen der Formel (I) hergestellt werden.

**Tabelle 3: Herstellungsbeispiele für die Verbindungen der Formel (I)**

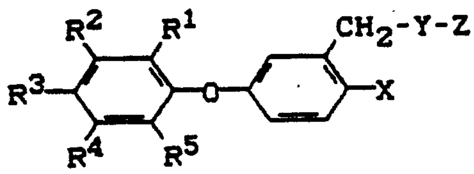

(I)

| Bsp.-Nr. | $R^1$ | $R^2$ | $R^3$ | $R^4$ | $R^5$ | X | Y | Z | Schmelzpunkt (°C) |
|---|---|---|---|---|---|---|---|---|---|
| 2 | Cl | H | $CF_3$ | H | Cl | Cl | O | $CO-CH_3$ | (δ=5,18)* |
| 3 | Cl | H | $CF_3$ | H | Cl | Cl | O | 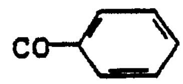 | |
| 4 | Cl | H | $CF_3$ | H | Cl | Cl | O | 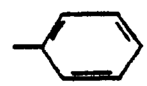 | |
| 5 | Cl | H | $CF_3$ | H | Cl | Cl | S | H | 123 |
| 6 | Cl | H | $CF_3$ | H | Cl | Cl | S | $CH_2CH_2OH$ | (δ=3,70)* |
| 7 | Cl | H | $CF_3$ | H | Cl | Cl | O | $CH(CH_3)COOC_2H_5$ | (δ=4,19)* |
| 8 | Cl | H | $CF_3$ | H | Cl | Cl | O | H | 116 |
| 9 | Cl | H | $CF_3$ | H | Cl | Cl | O | $CH_3$ | 64 |
| 10 | Cl | H | $CF_3$ | H | Cl | Cl | O | 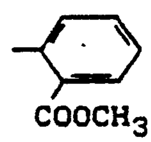 | 110 |

Tabelle 3 - Fortsetzung

| Bsp.-Nr. | $R^1$ | $R^2$ | $R^3$ | $R^4$ | $R^5$ | X | Y | Z | Schmelzpunkt (°C) |
|---|---|---|---|---|---|---|---|---|---|
| 11 | Cl | H | $CF_3$ | H | Cl | Cl | O | 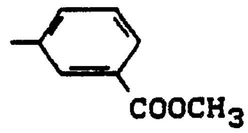 | 124 |
| 12 | Cl | H | $CF_3$ | H | Cl | Cl | O | 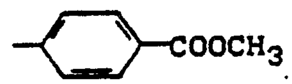 | 104 |
| 13 | Cl | H | $CF_3$ | H | Cl | Cl | O | 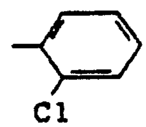 | 112 |
| 14 | Cl | H | $CF_3$ | H | Cl | Cl | O | 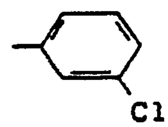 | 78 |
| 15 | Cl | H | $CF_3$ | H | Cl | Cl | O | 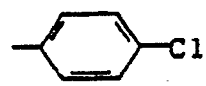 | 84 |
| 16 | Cl | H | $CF_3$ | H | Cl | Cl | O | 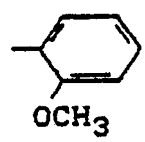 | 108 |
| 17 | Cl | H | $CF_3$ | H | Cl | Cl | O | 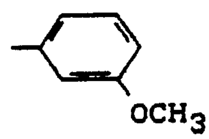 | 77 |
| 18 | Cl | H | $CF_3$ | H | Cl | Cl | O | 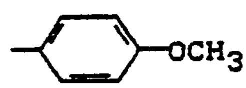 | ($\delta$=3,79)* |
| 19 | Cl | H | $CF_3$ | H | Cl | Cl | S | $CH_2COOCH_3$ | ($\delta$=3,79)* |
| 20 | Cl | H | $CF_3$ | H | Cl | Cl | O | $CH(CH_3)_2$ | ($\delta$=4,55)* |

<u>Tabelle 3</u> - Fortsetzung

| Bsp.-Nr. | $R^1$ | $R^2$ | $R^3$ | $R^4$ | $R^5$ | X | Y | Z | Schmelzpunkt (°C) |
|---|---|---|---|---|---|---|---|---|---|
| 21 | Cl | H | $CF_3$ | H | Cl | Cl | S | $CH_2COOH$ | 114 |
| 22 | Cl | H | $CF_3$ | H | Cl | Cl | O | $CO-CH_2COOCH_3$ | ($\delta$=5,25)* |
| 23 | Cl | H | $CF_3$ | H | Cl | Cl | S | $CH_2COOCHCOOCH_3$<br>\|<br>$CH_3$ | ($\delta$=3,70)* |
| 24 | Cl | H | $CF_3$ | H | Cl | Cl | S | $CH_2COOCH_2COOCH_3$ | ($\delta$=3,68)* |
| 25 | Cl | H | $CF_3$ | Cl | Cl | Cl | O | $COCH_3$ | 92 |
| 26 | Cl | H | $CF_3$ | Cl | Cl | Cl | S | $CH_2COOCH_3$ | 50 |
| 27 | Cl | H | $CF_3$ | H | Cl | Cl | O | $CO-CH(CH_3)_2$ | 74 |
| 28 | Cl | H | $CF_3$ | Cl | Cl | Cl | S | $CH_2COOC_2H_5$ | ($\delta$=3,79)* |
| 29 | Cl | H | $CF_3$ | H | Cl | Cl | S | $C-N(CH_3)_2$<br>‖<br>S | 110 |
| 30 | Cl | H | $CF_3$ | Cl | Cl | Cl | S | $CH_2COOH$ | ($\delta$=3,88)* |
| 31 | Cl | H | $CF_3$ | H | F | Cl | O | $COCH_3$ | ($\delta$=5,17)* |
| 32 | Cl | H | $CF_3$ | H | F | Cl | S | $CH_2CH_2OH$ | ($\delta$=3,80)* |
| 33 | Cl | H | $CF_3$ | H | F | Cl | O | $COCH(CH_3)_2$ | ($\delta$=5,17)* |
| 34 | Cl | H | $CF_3$ | H | F | Cl | S | $CH_2COOCH_3$ | 35 |
| 35 | Cl | H | $CF_3$ | H | Cl | Cl | S | $C-N(C_3H_7-i)_2$<br>‖<br>S | 70 |
| 36 | Cl | H | $CF_3$ | H | Cl | Cl | S | $CH_2CH_2OCOCH_3$ | |
| 37 | Cl | H | $CF_3$ | H | Cl | Cl | S | $CH_2CH_2OCOCH(CH_3)_2$ | |
| 38 | Cl | H | $CF_3$ | H | F | Cl | S | $CHCOOC_4H_9$<br>\|<br>$CH_3$ | |

<u>Tabelle 3</u> - Fortsetzung

| Bsp.-Nr. | $R^1$ | $R^2$ | $R^3$ | $R^4$ | $R^5$ | X | Y | Z | Schmelzpunkt (°C) |
|---|---|---|---|---|---|---|---|---|---|
| 39 | Cl | H | $CF_3$ | H | F | Cl | S | $CHCOOC_2H_5$<br>$CH_3$ | |
| 40 | Cl | H | $CF_3$ | H | F | Cl | SO | $CH_2COOCH_3$ | 82 |
| 41 | Cl | H | $CF_3$ | H | F | Cl | S | $CH_2CH_2COOC_2H_5$ | |
| 42 | Cl | H | $CF_3$ | H | Cl | Cl | S | $CHCOOC_3H_7$<br>$CH_3$ | |
| 43 | Cl | H | $CF_3$ | H | F | Cl | $SO_2$ | $CH_2COOCH_3$ | |
| 44 | Cl | H | $CF_3$ | H | F | Cl | SO | $CHCOOC_2H_5$<br>$CH_3$ | |
| 45 | Cl | H | $CF_3$ | H | F | Cl | SO | $CH_2CH_2COOC_2H_5$ | |
| 46 | Cl | H | $CF_3$ | H | F | Cl | S | $CH_2COOH$ | |
| 47 | Cl | H | $CF_3$ | H | F | Cl | S | $CHCOOH$<br>$CH_3$ | - |
| 48 | Cl | H | $CF_3$ | H | F | Cl | S | $CHCOOCH_3$<br>$CH_3$ | |
| 49 | Cl | H | $CF_3$ | H | F | Cl | S | $CH_2CH_2COOH$ | |
| 50 | Cl | H | $CF_3$ | H | F | Cl | S | $CH_2CH_2COOCH_3$ | 46 |
| 51 | Cl | H | $CF_3$ | H | Cl | Cl | S | $CHCOOC_2H_5$<br>$CH_3$ | |
| 52 | Cl | H | $CF_3$ | H | Cl | Cl | O | $CH_2CH_2COOCH_3$ | |
| 53 | Cl | H | $CF_3$ | H | Cl | Cl | S | $CHCOOH$<br>$CH_3$ | 99 |

Tabelle 3 - Fortsetzung

| Bsp.-Nr. | $R^1$ | $R^2$ | $R^3$ | $R^4$ | $R^5$ | X | Y | Z | Schmelzpunkt (°C) |
|---|---|---|---|---|---|---|---|---|---|
| 54 | Cl | H | $CF_3$ | H | Cl | Cl | S | $CH_2CH_2COOH$ | 85 |
| 55 | Cl | H | $CF_3$ | H | Cl | Cl | S | $CH_2CH_2COOCH_3$ | |
| 56 | Cl | H | $CF_3$ | H | Cl | Cl | S | $C-N(C_2H_5)_2$<br>‖<br>S | |
| 57 | Cl | H | $CF_3$ | H | Cl | Cl | S | $CHCOOCH_3$<br>│<br>$CH_3$ | |
| 58 | Cl | H | $CF_3$ | H | Cl | Cl | SO | $CH_2COOCH_3$ | |
| 59 | Cl | H | $CF_3$ | H | Cl | Cl | SO | $CH_2CH_2OH$ | 126 |
| 60 | Cl | H | $CF_3$ | H | Cl | Cl | S | $CH_2COOC_3H_7$ | |
| 61 | Cl | H | $CF_3$ | H | Cl | Cl | S | $CH_2COOCH(CH_3)_2$ | 68 |
| 62 | Cl | H | $CF_3$ | H | Cl | Cl | S | $CH_2COOC_4H_9$ | |
| 63 | Cl | H | $CF_3$ | H | Cl | Cl | S | $CH_2COOCH_2CH(CH_3)_2$ | |
| 64 | Cl | H | $CF_3$ | H | Cl | Cl | O | $CH_2COOCH_3$ | |
| 65 | Cl | H | $CF_3$ | H | Cl | Cl | O | $CHCOOCH_3$<br>│<br>$CH_3$ | |
| 66 | Cl | H | $CF_3$ | H | F | Cl | $SO_2$ | $CHCOOC_2H_5$<br>│<br>$CH_3$ | 96 |
| 67 | Cl | H | $CF_3$ | H | F | Cl | $SO_2$ | $CH_2CH_2COOC_2H_5$ | 100 |

*) Die $^1H$-NMR-Spektren wurden aufgenommen in $CDCl_3$ mit Tetramethylsilan als innerem Standard. Angegeben sind in der Regel die chemischen Verschiebungen als δ-Werte für die Gruppierung $-CH_2-$.

Die Herstellung der in Tabelle 3 als Beispiel 40 aufgeführten Verbindung ist nachsthend exemplarisch beschrieben.

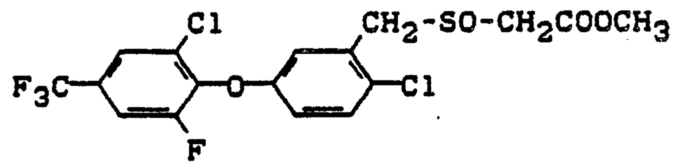

(Verfahren (c))

Eine Lösung von 1,6 g (9,3 mMol) 3-Chlor-perbenzoesäure in 30 ml Chloroform wird zu einer auf -10 °C gekühlten Lösung von 3,8 g (8,6 mMol) 5-(2-Chlor-5-(2-Chlor-6-fluor-4-trifluormethyl-phenoxy)-benzyl)-mercaptoessigsäure-methylester in 25 ml Chloroform unter Rühren tropfenweise gegeben. Das Reaktionsgemisch wird 3 Stunden bei -10 °C und 4 Stunden bei +5 °C gerührt. Zur Aufarbeitung wird mit 5%iger Natriumhydrogencarbonat-Lösung geschüttelt, die organische Phase abgetrennt, mit Magnesiumsulfat getrocknet und filtriert. Vom Filtrat wird das Lösungsmittel im Wasserstrahlvakuum sorgfältig abdestilliert. Man erhält 3,4 g (86 % der Theorie) 5-(2-Chlor-5-(2-chlor-6-fluor-4-trifluormethyl-phenoxy)-benzyl)-sulfinylessigsäuremethylester als kristallinen Rückstand vom Schmelzpunkt 82 °C.

Anwendungsbeispiele

In den folgenden Anwendungsbeispielen wird die nachstehend aufgeführte Verbindung als Vergleichssubstanz herangezogen:

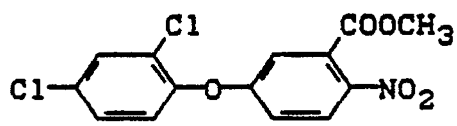

(A)

3-(2,4-Dichlor-phenoxy)-6-nitrobenzoesäure-methylester (bekannt aus US-PS 36 52 645 und US-PS 3 776 715).

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

| | | |
|---|---|---|
| 0 % | = | keine Wirkung (wie unbehandelte Kontrolle) |
| 100 % | = | totale Vernichtung |

In diesem Test zeigen beispielsweise die Verbindungen gemäß den Herstellungsbeispielen (2), (3) und

(4) bei guter Selektivität in Weizen erheblich stärkere Wirkung gegen Unkräuter, wie z. B. Datura, Portulak, Sinapis und Solanum, als die Vergleichssubstanz (A).

## Ansprüche

1. Halogensubstituierte Phenoxybenzyl(thi)ol-Derivate der allgemeinen Formel (I),

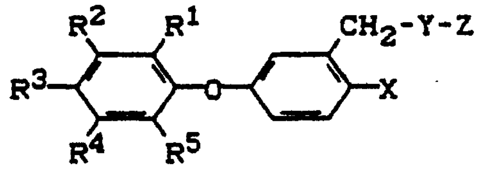

(I)

in welcher
$R^1$ für Wasserstoff, Halogen, Cyano oder Trifluormethyl steht,
$R^2$ für Wasserstoff oder Halogen steht,
$R^3$ für Halogen, Cyano, Trifluormethyl, Trifluormethoxy oder Trifluormethylsulfonyl steht,
$R^4$ für Wasserstoff oder Halogen steht,
$R^5$ für Wasserstoff oder Halogen steht,
-mit der Maßgabe, das wenigstens zwei der Substituenten $R^1$, $R^2$, $R^4$ und $R^5$ von Wasserstoff verschieden sind -
X für Halogen steht,
Y für Sauerstoff, Schwefel, Sulfinyl oder Sulfonyl steht und
Z für Wasserstoff, für gegebenenfalls durch Cyano, Halogen, Hydroxy, Alkoxy, Alkylthio, Carboxy, Alkoxycarbonyl, Alkylcarbonyloxy oder Alkoxycarbonylalkoxycarbonyl substituiertes Alkyl, für jeweils gegebenenfalls durch Halogen und/oder Alkyl substituiertes Cycloalkyl oder Cycloalkylalkyl, für jeweils gegebenenfalls durch Halogen substituiertes Alkenyl oder Alkinyl, oder für eine der nachstehenden Gruppierungen steht

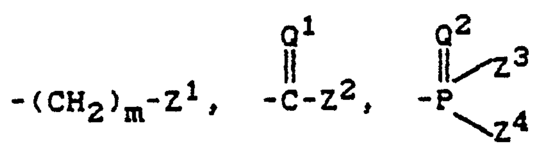

worin
m für die Zahlen 0, 1, 2 oder 3 steht,
$Q^1$ und $Q^2$ jeweils für Sauerstoff oder Schwefel stehen,
$Z^1$ für jeweils gegebenenfalls durch Halogen, Cyano, Nitro, Alkyl, Halogenalkyl, Alkoxy, Halogenalkoxy, Alkylthio, Halogenalkylthio, Dialkylamino, Carboxy und/oder Alkoxycarbonyl substituiertes Phenyl oder Naphthyl, oder für eine gegebenenfalls durch Halogen und/oder Alkyl substituierte heterocyclische Gruppierung aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl oder Pyrimidinyl steht,
$Z^2$ für Wasserstoff, für gegebenenfalls durch Halogen, Alkoxy oder Alkoxycarbonyl substituiertes Alkyl, für gegebenenfalls durch Halogen und/oder Alkyl substituiertes Cycloalkyl, für Alkenyl, Benzyl oder für gegebenenfalls durch Halogen, Cyano, Nitro, Alkyl, Halogenalkyl, Alkoxy, Halogenalkoxy, Alkylthio, Halogenalkylthio und/oder Alkoxycarbonyl substituiertes Phenyl, für Alkoxy, Benzyloxy, Phenoxy, Alkylthio, Benzylthio, Phenylthio oder Dialkylamino steht, und
$Z^3$ und $Z^4$ jeweils für gegebenenfalls durch Halogen substituiertes Alkyl oder Alkoxy stehen.

2. Verbindungen der allgemeinen Formel (I) gemäß Anspruch 1, dadurch gekennzeichnet, daß darin
$R^1$ für Wasserstoff, Fluor, Chlor, Brom, Cyano oder Trifluormethyl steht,
$R^2$ für Wasserstoff, Fluor, Chlor oder Brom steht,
$R^3$ für Fluor, Chlor, Brom, Cyano, Trifluormethyl, Trifluormethoxy oder Trifluormethylsulfonyl steht,
$R^4$ für Wasserstoff, Fluor, Chlor oder Brom steht,
$R^5$ für Wasserstoff, Fluor, Chlor oder Brom steht

- mit der Maßgabe, daß wenigstens zwei der Substituenten $R^1$, $R^2$, $R^4$ und $R^5$ von Wasserstoff verschieden sind -
X für Fluor, Chlor oder Brom steht,
Y für Sauerstoff, Schwefel, Sulfinyl oder Sulfonyl steht und
Z für Wasserstoff, für gegebenenfalls durch Cyano, Fluor, Chlor, Brom, Hydroxy, $C_1$-$C_6$-Alkoxy, $C_1$-$C_6$-Alkylthio, Carboxy, $C_1$-$C_6$-Alkoxy-carbonyl, $C_1$-$C_6$-Alkyl-carbonyloxy oder $C_1$-$C_6$-Alkoxy-carbonyl-$C_1$-$C_6$-alkoxy-carbonyl substituiertes $C_1$-$C_6$-Alkyl, für jeweils gegebenenfalls durch Fluor, Chlor, Brom und/oder $C_1$-$C_4$-Alkyl substituiertes $C_3$-$C_8$-Cycloalkyl oder $C_3$-$C_8$-Cycloalkyl-$C_1$-$C_4$-alkyl, für jeweils gegebenenfalls durch Fluor, Chlor und/oder Brom substituiertes $C_3$-$C_6$-Alkenyl oder $C_3$-$C_6$-Alkinyl, oder für eine der nachstehenden Gruppierungen steht

$-(CH_2)_m-Z^1$, 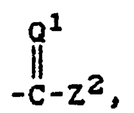, 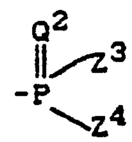

worin
m für die Zahlen 0, 1, 2 oder 3 steht,
$Q^1$ und $Q^2$ jeweils für Sauerstoff oder Schwefel stehen,
$Z^1$ für jeweils gegebenenfalls durch Fluor, Chlor, Brom, Iod, Cyano, Nitro, $C_1$-$C_4$-Alkyl, fluorsubstituiertes und/oder chlorsubstituiertes $C_1$-$C_2$-Alkyl, $C_1$-$C_4$-Alkoxy, fluorsubstituiertes und/oder chlorsubstituiertes $C_1$-$C_2$-Alkoxy, $C_1$-$C_4$-Alkylthio, fluorsubstituiertes und/oder chlorsubstituiertes $C_1$-$C_2$-Alkylthio, Di-($C_1$-$C_2$-alkyl)-amino, Carboxy und/oder $C_1$-$C_4$-Alkoxy-carbonyl substituiertes Phenyl oder Naphthyl, oder für eine gegebenenfalls durch Fluor, Chlor, Brom und/oder $C_1$-$C_4$-Alkyl substituierte heterocyclische Gruppierung aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl oder Pyrimidinyl steht,
$Z^2$ für Wasserstoff, für gegebenenfalls durch Fluor, Chlor, Brom, $C_1$-$C_4$-Alkoxy oder $C_1$-$C_4$-Alkoxy-carbonyl substituiertes $C_1$-$C_6$-Alkyl, für gegebenenfalls durch Fluor, Chlor, Brom und/ oder $C_1$-$C_4$-Alkyl substituiertes $C_3$-$C_6$-Cycloalkyl, für $C_3$-$C_6$-Alkenyl, Benzyl oder für gegebenenfalls durch Fluor, Chlor, Brom, Iod, Cyano, Nitro, $C_1$-$C_4$-Alkyl, fluorsubstituiertes und/oder chlorsubstituiertes $C_1$-$C_2$-Alkyl, $C_1$-$C_4$-Alkoxy, fluorsubstituiertes und/oder chlor substituiertes $C_1$-$C_2$-Alkoxy, $C_1$-$C_4$-Alkylthio, fluorsubstituiertes und/oder chlorsubstituiertes $C_1$-$C_2$-Alkylthio und/oder $C_1$-$C_4$-Alkoxy-carbonyl substituiertes Phenyl, für $C_1$-$C_4$-Alkoxy, Benzyloxy, Phenoxy, $C_1$-$C_4$-Alkylthio, Benzylthio, Phenylthio oder Di-($C_1$-$C_4$-alkyl)-amino steht und
$Z^3$ und $Z^4$ jeweils für gegebenenfalls durch Fluor und/oder Chlor substituiertes $C_1$-$C_4$-Alkyl oder $C_1$-$C_4$-Alkoxy stehen.

3. Verbindungen der allgemeinen Formel (I) gemäß Anspruch 1, dadurch gekennzeichnet, daß darin
$R^1$ für Chlor steht,
$R^2$ für Wasserstoff steht,
$R^3$ für Trifluormethyl steht,
$R^4$ für Wasserstoff, Fluor oder Chlor steht,
$R^5$ für Fluor oder Chlor steht,
X für Chlor oder Brom steht,
Y für Sauerstoff, Schwefel, Sulfinyl oder Sulfonyl steht und
Z für Wasserstoff, für gegebenenfalls durch Cyano, Fluor, Chlor, Hydroxy, $C_1$-$C_4$-Alkoxy, $C_1$-$C_4$-Alkylthio, Carboxy, $C_1$-$C_4$-Alkoxy-carbonyl, $C_1$-$C_4$-Alkyl-carbonyloxy oder $C_1$-$C_4$-Alkoxy-carbonyl-$C_1$-$C_2$-alkoxy-carbonyl substituiertes $C_1$-$C_4$-Alkyl, für jeweils gegebenenfalls durch Chlor und/oder Methyl substituiertes $C_3$-$C_6$-Cycloalkyl oder $C_3$-$C_6$-Cycloalkyl-$C_1$-$C_2$-alkyl, für jeweils gegebenenfalls durch Chlor substituiertes $C_3$-$C_4$-Alkenyl oder $C_3$-$C_4$-Alkinyl, oder für eine der nachstehenden Gruppierungen steht

$-(CH_2)_m-Z^1$, 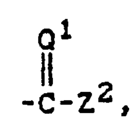, 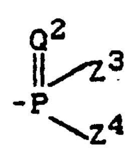

worin
m für die Zahlen 0, 1 oder 2 steht,
$Q^1$ und $Q^2$ jeweils für Sauerstoff oder Schwefel stehen,
$Z^1$ für jeweils gegebenenfalls durch Fluor, Chlor, Brom, Cyano, Nitro, Methyl, Ethyl, Trifluormethyl, Methoxy, Ethoxy, Difluormethoxy, Trifluormethoxy, Methylthio, Ethylthio, Difluormethylthio, Trifluormethylthio, Dimethylamino und/oder Methoxycarbonyl substituiertes Phenyl oder Naphthyl, oder für eine gegebenenfalls durch Chlor und/oder Methyl substituierte heterocyclische Gruppierung aus der Reihe Furyl, Tetrahydrofuryl, Thienyl, Perhydropyranyl, Oxazolyl, Thiazolyl oder Dioxolanyl steht,
$Z^2$ für Wasserstoff, für gegebenenfalls durch Fluor, Chlor, $C_1$-$C_2$-Alkoxy oder $C_1$-$C_2$-Alkoxycarbonyl substituiertes $C_1$-$C_4$-Alkyl, für gegebenenfalls durch Chlor und/oder Methyl substituiertes $C_3$-$C_6$-Cycloalkyl, für $C_3$-$C_4$-Alkenyl, Benzyl, für gegebenenfalls durch Fluor, Chlor, Brom, Nitro, Methyl, Ethyl, Trifluormethyl, Methoxy, Ethoxy, Difluormethoxy, Trifluormethoxy und/oder Methoxycarbonyl substituiertes Phenyl oder für Dimethylamino steht, und
$Z^3$ und $Z^4$ jeweils für $C_1$-$C_3$-Alkoxy stehen.

4. Verfahren zur Herstellung von halogensubstituierten Phenoxybenzyl(thio)ol-Derivaten der allgemeinen Formel (I)

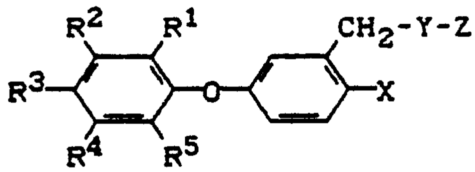 (I)

in welcher
$R^1$ für Wasserstoff, Halogen, Cyano oder Trifluormethyl steht,
$R^2$ für Wasserstoff oder Halogen steht,
$R^3$ für Halogen, Cyano, Trifluormethyl, Trifluormethoxy oder Trifluormethylsulfonyl steht,
$R^4$ für Wasserstoff oder Halogen steht,
$R^5$ für Wasserstoff oder Halogen steht,
- mit der Maßgabe, das wenigstens zwei der Substituenten $R^1$, $R^2$, $R^4$ und $R^5$ von Wasserstoff verschieden sind -
X für Halogen steht,
Y für Sauerstoff, Schwefel, Sulfinyl oder Sulfonyl steht und
Z für Wasserstoff, für gegebenenfalls durch Cyano, Halogen, Hydroxy, Alkoxy, Alkylthio, Carboxy, Alkoxycarbonyl, Alkylcarbonyloxy oder Alkoxycarbonylalkoxycarbonyl substituiertes Alkyl, für jeweils gegebenenfalls durch Halogen und/oder Alkyl substituiertes Cycloalkyl oder Cycloalkylalkyl, für jeweils gegebenenfalls durch Halogen substituiertes Alkenyl oder Alkinyl, oder für eine der nachstehenden Gruppierungen steht

$-(CH_2)_m-Z^1$, 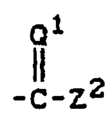, 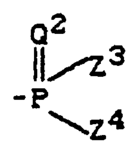

worin
m für die Zahlen 0, 1, 2 oder 3 steht,
$Q^1$ und $Q^2$ jeweils für Sauerstoff oder Schwefel stehen,
$Z^1$ für jeweils gegebenenfalls durch Halogen, Cyano, Nitro, Alkyl, Halogenalkyl, Alkoxy, Halogenalkoxy, Alkylthio, Halogenalkylthio, Dialkylamino, Carboxy und/oder Alkoxycarbonyl substituiertes Phenyl oder Naphthyl, oder für eine gegebenenfalls durch Halogen und/oder Alkyl substituierte heterocyclische Gruppierung aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl oder Pyrimidinyl steht,
$Z^2$ für Wasserstoff, für gegebenenfalls durch Halogen, Alkoxy oder Alkoxycarbonyl substituiertes Alkyl, für gegebenenfalls durch Halogen und/oder Alkyl substituiertes Cycloalkyl, für Alkenyl, Benzyl oder für gegebenenfalls durch Halogen, Cyano, Nitro, Alkyl, Halogenalkyl, Alkoxy, Halogenalkoxy, Alkylthio, Halogenalkylthio und/oder Alkoxycarbonyl substituiertes Phenyl, für Alkoxy, Benzyloxy, Phenoxy, Alkylthio, Benzylthio, Phenylthio oder Dialkylamino steht, und
$Z^3$ und $Z^4$ jeweils für gegebenenfalls durch Halogen substituiertes Alkyl oder Alkoxy stehen.
dadurch gekennzeichnet, daß man
(a) halogensubstituierte Phenoxybenzylhalogenide der allgemeinen Formel (II)

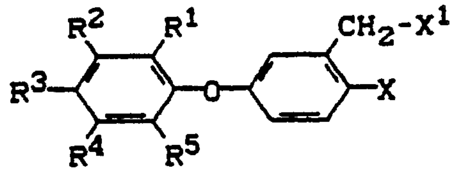
(II)

in welcher
$R^1$, $R^2$, $R^3$, $R^4$, $R^5$ und X die in Anspruch 1 angegebenen Bedeutungen haben und
$X^1$ für Halogen steht,
mit nucleophilen Verbindungen der allgemeinen Formel (III)
M - Y - Z (III)
in welcher
Y und Z die oben angegebenen Bedeutungen haben und
M für Wasserstoff, Natrium oder Kalium steht,
gegebenenfalls in Gegenwart von Säureakzeptoren und gegebenenfalls in Gegenwart von Verdünnungsmitteln umsetzt, oder daß man
(b) halogensubstituierte Phenoxybenzyl(thi)ole der allgemeinen Formel (Ia)

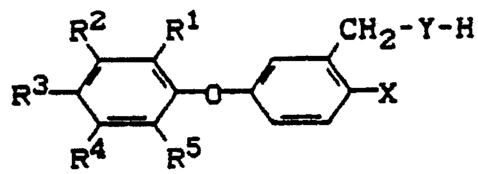
(Ia)

in welcher
$R^1$, $R^2$, $R^3$, $R^4$, $R^5$, X und Y die in Anspruch 1 angegebenen Bedeutungen haben,
mit elektrophilen Verbindungen der allgemeinen Formel (IV)
$X^2$ - Z (IV)
in welcher
Z die in Anspruch 1 angegebene Bedeutung hat und
$X^2$ für eine nucleophile Abgangsgruppe steht,
gegebenenfalls in Gegenwart von Säureakzeptoren und gegebenenfalls in Gegenwart von Verdünnungsmitteln umsetzt, oder daß man
(c) halogensubstituierte Phenoxybenzylthiol-Derivate der allgemeinen Formel (Ib)

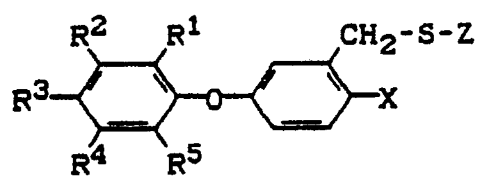
(Ib)

in welcher
$R^1$, $R^2$, $R^3$, $R^4$, $R^5$, X und Zdie oben angegebenen Bedeutungen haben,
mit Oxidationsmitteln, gegebenenfalls in Gegenwart von Verdünnungsmitteln umsetzt.

5. Herbizide Mittel, gekennzeichnet durch einen Gehalt an mindestens einem halogensubstituierten Phenoxybenzyl(thi)ol-Derivate der Formel (I) gemäß den Ansprüchen 1 bis 4.

6. Verfahren zur Bekämpfung von Unkräutern, dadurch gekennzeichnet, daß man halogensubstituierte Phenoxybenzyl(thi)ol-Derivate der Formel (I) gemäß den Ansprüchen 1 bis 4 auf die Unkräuter und/oder ihren Lebensraum einwirken läßt.

7. Verwendung von halogensubstituierte Phenoxybenzyl(thi)ol-Derivate der Formel (I) gemäß den Ansprüchen 1 bis 4 zur Bekämpfung von Unkräutern.

8. Verfahren zur Herstellung von herbiziden Mitteln, dadurch gekennzeichnet, daß man halogensubstituierte Phenoxybenzyl(thi)ol-Derivate der Formel (I) gemäß den Ansprüchen 1 bis 4 mit Streckmitteln und/oder oberflächenaktiven Substanzen vermischt.

Europäisches Patentamt

# EUROPÄISCHER RECHERCHENBERICHT

Nummer der Anmeldung

EP 89108066.5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP - A1 - 0 056 119 (BASF) * Ansprüche * -- | 1-8 | C 07 C 149/273<br>C 07 C 43/29<br>C 07 C 43/295<br>C 07 C 59/68<br>C 07 C 69/736<br>C 07 C 155/08<br>C 07 C 147/00<br>A 01 N 31/00 |
| X | EP - A1 - 0 023 890 (CIBA-GEIGY) * Anspruch 1; Seiten 5,11 * -- | 1-3 | |
| P,A | US - A - 4 788 348 (ALAN J.WHITTLE) * Ansprüche * -- | 1 | |
| A | DE - A1 - 3 139 976 (MITSUI TOATSU CHEMICALS) * Ansprüche 1,9 * -- | 1,4 | |
| A | EP - A2 - 0 077 961 (BAYER) * Ansprüche 1,3-6 * -- | 1,4-8 | **RECHERCHIERTE SACHGEBIETE (Int. Cl.4)** |
| A | FR - A - 2 351 096 (BAYER) * Anspruch 1 * -- | 1 | C 07 C 43/00<br>C 07 C 59/00<br>C 07 C 69/00<br>C 07 C 149/00<br>C 07 C 147/00 |
| A | DE - A1 - 2 933 985 (BAYER) * Anspruch 1 * -- | 1 | |
| A | AT - B - 322 278 (SHELL) * Anspruch 1; Tabelle 1, letzte Verbindung * ---- | 1 | |

Der vorliegende Recherchenbericht wurde für alle Patentansprüche erstellt.

| Recherchenort | Abschlußdatum der Recherche | Prüfer |
|---|---|---|
| WIEN | 20-07-1989 | REIF |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument